Europäisches Patentamt

European Patent Office

Office européen des brevets

(11) Publication number: **0 067 377**
A2

# (12) EUROPEAN PATENT APPLICATION

(21) Application number: 82104895.6

(22) Date of filing: 03.06.82

(51) Int. Cl.³: **A 61 F 13/16**

(30) Priority: 11.06.81 US 272773

(43) Date of publication of application:
22.12.82 Bulletin 82/51

(84) Designated Contracting States:
BE DE FR GB LU NL

(71) Applicant: KIMBERLY-CLARK CORPORATION
North Lake Street
Neenah Wisconsin 54956(US)

(72) Inventor: Neuwirth, Joseph G.
920 Chancelant Court
Neenah, Wisconsin 54956(US)

(74) Representative: Patentanwälte Grünecker, Dr.
Kinkeldey, Dr. Stockmair, Dr. Schumann, Jakob, Dr.
Bezold, Meister, Hilgers, Dr. Meyer-Plath
Maximilianstrasse 43
D-8000 München 22(DE)

(54) Sanitary napkin prestressed in lateral direction.

(57) A sanitary napkin is transversely prestressed in the crotch area to compressively set the napkin in a configuration having reduced width in that area.

FIG. 2

EP 0 067 377 A2

Croydon Printing Company Ltd

## FIELD OF THE INVENTION

The subject invention relates to a sanitary napkin, particularly to a sanitary napkin having reduced width in the crotch area.

## BACKGROUND OF THE INVENTION

One of the desirable attributes of the sanitary napkin is comfort. Toward this end, a variety of materials have been utilized, and a multitude of configurations tried in attempts to provide a sanitary napkin which can be comfortably worn for extended periods. Among the variety of configurations utilized in an attempt to increase the comfort of a napkin is one in which the napkin is folded in its leading edge and tapers to an area of increasing width from the front to back portion. Such a napkin is disclosed in U. S. Patent Nos. 4,067,336 and 4,182,334 issued to Russ Johnson.

Another attempt at producing a more comfortable pad by shape alteration has resulted in a pad in which inwardly directed curvilinear areas are cut out in the portion of the pad adjacent the crotch. The idea behind these cutouts is to produce a napkin in which the edge shape in the crotch area conforms generally to the shape of the upper thighs of the wearer. Cut out napkins of this type, however, tend to chafe at the edges. This is true because the absorbent material and the fluid impervious baffle are coterminous and sealed at or near the lateral edges. The coterminous edges of the baffle and the absorbent provide rough surfaces and movement of the wearer exacerbates the chafing effects of these edges.

Other shape conforming approaches are disclosed, for example, in U. S. Patent 2,064,431 in which a napkin has an inwardly and upwardly folded central portion in the crotch area and raised portions at either end. Also, U. S. Patent 2,549,982 describes a diaper in which a gauze outer wrap is tightened to constrict and restrain the absorbent material in the central portion. Also see U. S. Patent 3,805,790 for another example of a tapered pad.

Interviews with several napkin wearers revealed that, regardless of the particular napkin chosen, whether the napkin is full sized or of the panty liner type, the napkin wearer undertakes a series of movements to position the napkin comfortably. These series of movements inevitably fold or crush the napkin in the portion surrounding the perineal area of the wearer, which when referring to the corresponding area in a napkin will hereinafter be referred to as the crotch portion. Evidently, therefore, there are two comfort criteria associated with wearing of a sanitary napkin. The first is associated with the napkin as it feels initially and, the second is the comfort attained when the napkin is distorted by the wearers movements. This invention provides a napkin which is responsive to this second comfort parameter.

## SUMMARY OF THE INVENTION

This invention provides a sanitary napkin in which the sides of the napkin in the crotch area are compressively prestressed to provide increased comfort to the wearer.

Sanitary napkins conventionally have a fluid impervious baffle and an absorbent layer. The absorbent layer is conventionally made of cellulosic materials such as wood pulp fluff and the like and may or may not contain thermoplastic fibers to increase resiliency and maintain open capillaries for fluid transmission through the cellulosic absorbent. In other configurations, multiple layers of absorbents are used in which a predominantly cellulosic layer is employed as the main absorbent reservoir in combination with additional layers which contain thermoplastic material and designed primarily to add resilience and conduct fluid away from the top surface of the napkin. All of these configurations may be employed to produce the prestressed napkin of this invention.

When layers of cellulosic material, thermoplastic fibrous material or mixtures of the two are subjected to sufficient compressive forces and these forces are released, the material will not return completely to its original configuration along the vectors in which the force was applied. In other words, some of the deformation applied on this flexible material by these forces will be permanent. This lateral deformation associated with the compressive forces is applied by the legs of the wearer of a sanitary napkin. The sanitary napkin of this invention is prestressed to provide a compressive set in the crotch area so that the second comfort parameter discussed above is at least partially satisfied without the wearer having to exert these compressive setting forces to prop-

The compressive set applied during the manufacture of this napkin is not designed to eliminate all of the compression of the napkin during use. In order to provide the resiliency necessary for comfort, the napkin must still be laterally compressible by the wearer. A compression of at least 10% of the width of the napkin in the crotch area is necessary to attain the comfort parameters according to this invention. Compression levels of 25 to 33% are generally preferred.

The compressive set desirable varies with the general napkin configuration and its initial width in the crotch area but the 25 to 33% compressive levels for the compressive set has been found to be a generally acceptable range. A compressive set in which the width in the crotch area is less than 45 millimeters substantially eliminates the resilience necessary for further deformation and is therefore to be avoided.

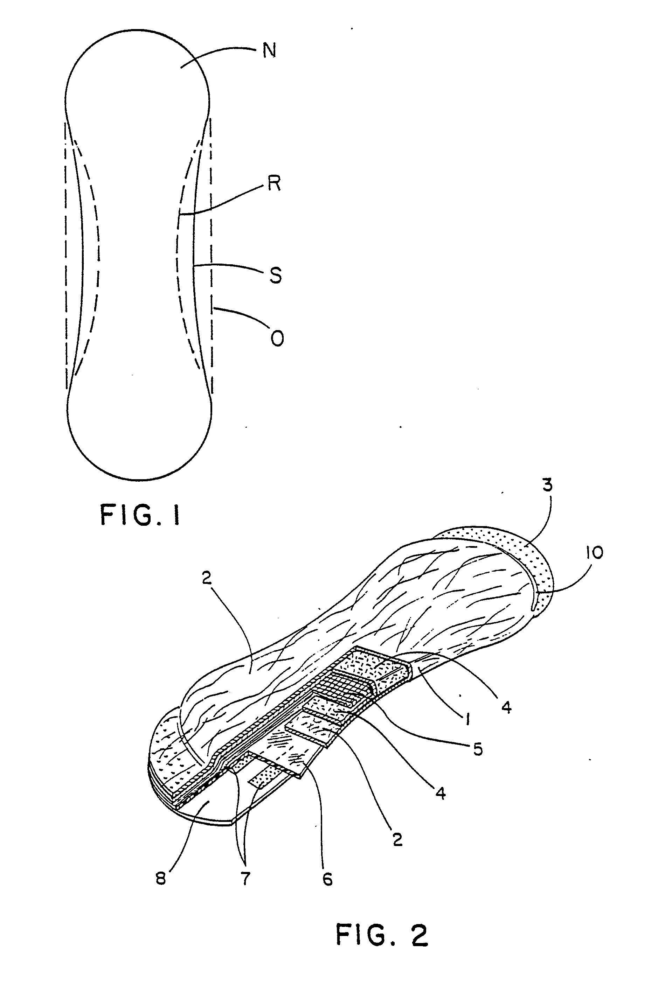

The invention may be more readily understood by reference to the drawings in which FIG. 1 is a pictorial representation of a plan view of a napkin showing compressive set and resiliency lines and FIG. 2 is a particularly preferred embodiment of a napkin according to this invention. As can be seen in FIG. 1, the napkin N has assumed a configuration in the crotch area represented by solid lines S after being subjected to compressive set forces. The outward dotted lines O present the original napkin configuration and the inward dotted lines R show the remaining deformation possible resulting from the resiliency remaining in the napkin and after compressive forces are applied by the wearer during use. The distance between lines R and S is an elastic distance and when the lateral compression of the napkin provided by the wearer during movement is shifted the sides of the napkin will move inwardly and outwardly as needed to conform to these movements. Although there may be additional permanent deformation as the absorbent becomes saturated due to the collapsing of the absorbent material, this will only occur to a minor extent.

According to FIG. 2, a napkin having folded edges which have been subject to deformation illustrated by sides 1 is provided according to the teachings of this invention  The napkin is assembled by placing layers of absorbent 4 and cover 2 to a support 5 and folding the sides of the napkin over to form a double thickness of each of these layers, applying a baffle 6 to provide a fluid impervious layer attaching adhesive strip 7 to the baffle along with a release liner 8 to prevent the premature adhesion of the strip 7. The ends of the napkin are sealed at line 10 either by fusing e.g. ultrasonically or by adhesive means leaving free ends 3.

When the teachings of this invention are employed to deform a napkin having folded edges, an especially comfortable nonchafing shape conforming surface results and the embodiment depicted in FIG. 2 is preferred for that reason although the concept of the invention applies for napkins of any configuration or construction.

Compressive set during manufacture can be obtained by a variety of means which are related to the construction of the particular napkin. Variables in composition that affect the compressive set are fluff

density, moisture and width in the crotch area. The greater the fluff density, the more force will be needed to provide compressive set. If the absorbent layer is primarily cellulosic, and if moisture is present within the cellulosic matrix, less force will be needed to attain permanent deformation. Obviously, greater force will be needed to obtain the same degree of set for napkins which are wider in the crotch area. (It should be noted that for purposes of this invention, the parameters set forth for the crotch area refer to the narrowest portion of the crotch after deformation.)

Compressive set per se can be accomplished by subjecting the sides of the napkin to a single deformation, reciprocating plungers or a series of deformations or, by the application of heat.

The utilization of heat energy in combination with steady side compressive force is currently preferred as being the most rapid means for attaining compressive set. One method particularly promising is the utilization of microwave energy to heat set the napkin while the napkin is in its deformed position. The presence of fusible fibers will obviously be an aid to setting by heat or microwave energy.

WHAT IS CLAIMED IS:

1.  A sanitary napkin having a fluid impervious baffle and an absorbent layer, said napkin being transversely compressively set in the crotch area to a width less than the width of the napkin in its uncompressed condition.

2.  The napkin according to Claim 1 wherein the width in the crotch area after compressive setting is at least 10% less than the width prior to compressive setting.

3.  The napkin according to Claim 1 wherein the width in the crotch area after compressive setting is at least 45 mm.

4.  The napkin according to Claims 1, 2, 3 or 6 wherein the absorbent layer is primarily cellulosic.

5.  The napkin according to Claims 1, 2, 3 or 6 wherein the absorbent layer contains fusible fibers.

6.  The napkin according to Claim 1 wherein the sides of the absorbent layer are folded.

0067377

FIG. 1

FIG. 2